# EUROPÄISCHE PATENTANMELDUNG

(11) **EP 4 725 429 A1**
(43) Veröffentlichungstag der Anmeldung: **15.04.2026**
(21) Anmeldenummer: 24205733.9
(22) Anmeldetag: 10.10.2024
(51) Int. Cl.: A61B 17/221, A61B 17/50

(54) **FREMDKÖRPERBERGEEINRICHTUNG FÜR EINE ENDOSKOPISCHE VORRICHTUNG, ENDOSKOPISCHE VORRICHTUNG SOWIE VERFAHREN ZUR HERSTELLUNG EINER FREMDKÖRPERBERGEEINRICHTUNG**

(71) Anmelder: FUJIFILM Corporation, Tokyo 107-0052 (JP)
(72) Erfinder: TSYRULNYKOV, Anton, 90431 Nürnberg (DE)
(74) Vertreter: Gosdin, Carstensen & Partner Patentanwälte Partnerschaftgesellschaft mbB

(57) **Zusammenfassung**

Die Erfindung betrifft eine Fremdkörperbergeeinrichtung (1) für eine endoskopische Vorrichtung, umfassend einen Bergeabschnitt (2), der ein Bergenetz (5) bildet, und einen hieran angrenzenden Befestigungsabschnitt (3), welcher für eine Befestigung der Fremdkörperbergeeinrichtung (1) in der endoskopischen Vorrichtung vorgesehen ist. Zumindest das Bergenetz (5) des Bergeabschnitts (2) und der Befestigungsabschnitt (3) sind gemeinsam einstückig als textiles Gebilde (9) ausgestaltet, welches durch Verknüpfen mindestens eines Filaments (10) hergestellt ist. Dabei bildet das Gebilde (9) bei dem Befestigungsabschnitt (3) mindestens eine Montageöse (7), an welcher die Befestigung der Fremdkörperbergeeinrichtung (1) in der endoskopischen Vorrichtung herzustellen ist.

## Beschreibung

Die Erfindung betrifft eine Fremdkörperbergeeinrichtung für eine endoskopische Vorrichtung, umfassend einen Bergeabschnitt, der ein Bergenetz bildet, und einen hieran angrenzenden Befestigungsabschnitt, welcher für eine Befestigung der Fremdkörperbergeeinrichtung in der endoskopischen Vorrichtung vorgesehen ist, wobei zumindest das Bergenetz des Bergeabschnitts und der Befestigungsabschnitt gemeinsam einstückig als textiles Gebilde ausgestaltet sind, welches durch Verknüpfen mindestens eines Filaments hergestellt ist. Des Weiteren betrifft die Erfindung eine endoskopische Vorrichtung sowie ein Verfahren zur Herstellung einer Fremdkörperbergeeinrichtung.

Fremdkörperbergeeinrichtungen werden im endoskopischen Bereich beispielsweise zum Entfernen von Fremdkörpern aus dem Gastrointestinaltrakt verwendet, wobei es sich bei einem zu entfernenden Fremdkörper um einen von dem jeweiligen Patienten verschluckten Körper oder auch um medizinische Hilfsmittel, wie z.B. Stents, Sonden usw. handeln kann. Zudem werden auch abgetragene Polypen über endoskopische Fremdkörperbergeeinrichtungen geborgen. Neben Fremdkörperzangen und Greifern kommen hierbei auch Fremdkörperbergeeinrichtungen mit Bergenetzen zum Einsatz. Bergenetze haben den Vorteil, dass neben einem einfachen intuitiven Handling auch ein sicherer Einschluss des Bergeobjekts während der gastroösophagealen Passage mit dem minimalen Risiko der Aspiration bei Verlust realisierbar ist. Dabei wird ein Bergenetz zumeist in Gastroskopen bzw. Enteroskopen verwendet, welche bei Kindern auch sehr dünn ausgeführt sein können.

Eine ein Bergenetz aufweisende Fremdkörperbergeeinrichtung ist beim Einführen des Endoskops üblicherweise vollständig in einen Tubus eingezogen und wird zur Bergung des jeweiligen Fremdkörpers über einen Handteil ausgeschoben. Teilweise ist das Bergenetz an einer Schlaufe befestigt, welche durch ein drahtförmiges Element gebildet wird, wobei sich die Schlaufe beim Ausschieben eines Zugseiles oder -drahtes des Endoskops vorzugsweise kreisförmig, elliptisch oder oval, aufweitet, wodurch das Netz dann in der Folge aufgespannt wird. Hierbei ist stets zu gewährleisten, dass das Bergenetz zuverlässig seitens der endoskopischen Vorrichtung befestigt ist.

Aus der US 11,871,957 B2 geht eine Fremdkörperbergeeinrichtung hervor, die einen Bergeabschnitt und einen hieran angrenzenden Befestigungsabschnitt aufweist. Während der blattähnlich gestaltete Bergeabschnitt ein Bergennetz der Fremdkörperbergeeinrichtung bildet, dient der Befestigungsabschnitt einer Befestigung der Fremdkörperbergeeinrichtung in einer endoskopischen Vorrichtung. Dazu weist der Befestigungsabschnitt eine rechteckförmige Gestalt auf, wobei der Befestigungsabschnitt bei der Befestigung der Fremdkörperbergeeinrichtung um den zugehörigen Anschlusspunkt in der endoskopischen Vorrichtung gewickelt und festgeknotet wird. Der Bergeabschnitt und der Befestigungsabschnitt sind einstückig aus Filament hergestellt, welches zu Maschen verschlungen und hierdurch ein den Bergeabschnitt und den Befestigungsabschnitt bildendes, textiles Gebilde realisiert.

Ausgehend vom vorstehend beschriebenen Stand der Technik ist es nun die Aufgabe der vorliegenden Erfindung eine Fremdkörperbergeeinrichtung zu schaffen, welche sich durch einen möglichst niedrigen Herstellungsaufwand auszeichnet und bei der gleichzeitig eine zuverlässige Befestigung innerhalb einer endoskopischen Vorrichtung möglich ist.

Diese Aufgabe wird aus vorrichtungstechnischer Sicht ausgehend vom Oberbegriff des nebengeordneten Anspruchs 1 in Verbindung mit dessen kennzeichnenden Merkmalen gelöst. Die hierauf folgenden, abhängigen Ansprüche geben jeweils vorteilhafte Weiterbildungen der Erfindung wieder. Eine endoskopische Vorrichtung, in welcher eine erfindungsgemäße Fremdkörperbergeeinrichtung vorgesehen ist, ist ferner Gegenstand des Anspruchs 11. Zudem erfolgt noch eine Lösung der Aufgabe aus verfahrenstechnischer Sicht ausgehend vom Oberbegriff des nebengeordneten Anspruchs 12 in Verbindung mit dessen kennzeichnenden Merkmalen, wobei die Ansprüche 13 bis 15 jeweils vorteilhafte Weiterbildungen der Erfindung wiedergeben.

Gemäß dem nebengeordneten Anspruch 1 umfasst eine Fremdkörperbergeeinrichtung einen Bergeabschnitt, der ein Bergenetz bildet, und einen hieran angrenzenden Befestigungsabschnitt, welcher für eine Befestigung der Fremdkörperbergeeinrichtung in einer endoskopischen Vorrichtung vorgesehen ist. Dabei sind zumindest das Bergenetz des Bergeabschnitts und der Befestigungsabschnitt gemeinsam einstückig als textiles Gebilde ausgestaltet sind, welches durch Verknüpfen mindestens eines Filaments hergestellt ist.

Die erfindungsgemäße Fremdkörperbergeeinrichtung weist also einen Bergeabschnitt und einen Befestigungsabschnitt auf, wobei der Bergeabschnitt und der Befestigungsabschnitt hierbei aneinander angrenzen, d.h. unmittelbar benachbart zueinander ausgestaltet sind. Bei dem Bergeabschnitt der Fremdkörperbergeeinrichtung ist dabei ein Bergenetz definiert, welches bei Anwendung der Fremdkörperbergeeinrichtung in einer endoskopischen Vorrichtung eine Aufnahme eines zu bergenden Fremdkörpers dient. Bevorzugt weist das Bergenetz in einem aufgespannten Zustand, welchen das Bergenetz bei Einsatz der Fremdkörperbergeeinrichtung zum Bergen eines Fremdkörpers einnimmt, eine bauchige Gestalt auf. Dadurch wird durch das Bergenetz ein Volumen zur Aufnahme des zu bergenden Fremdkörpers gebildet, wodurch eine Aufnahme und ein Umschließen des Fremdkörpers bei Einsatz der Fremdkörperbergeeinrichtung für eine Bedienperson der endoskopischen Vorrichtung vereinfacht wird. Der Bergeabschnitt weist insbesondere eine blattähnliche Gestalt auf, die bevorzugt durch eine Außenkontur des Bergeabschnitts definiert ist. Hierdurch wird alternativ, bevorzugt aber ergänzend zu der bauchigen Gestaltung des Bergenetzes ein Aufbau des Bergeabschnitts verwirklicht, bei welchem das Handling der Fremdkörperbergeeinrichtung bei Anwendung in einer endoskopischen Vorrichtung vereinfacht ist.

Der Befestigungsabschnitt ist bei der erfindungsgemäßen Fremdkörperbergeeinrichtung für eine Befestigung der Fremdkörperbergeeinrichtung in der endoskopischen Vorrichtung vorgesehen. Insofern wird also an dem Befestigungsabschnitt bei Anwendung der Fremdkörperbergeeinrichtung in einer endoskopischen Vorrichtung die Befestigung der Fremdkörperbergeeinrichtung seitens der restlichen endoskopischen Vorrichtung vorgenommen und in der Folge auch Betätigungsbewegungen an dem Befestigungsabschnitt in die Fremdkörperbergeeinrichtung eingeleitet. Besonders bevorzugt dient der Befestigungsabschnitt dabei als einzige Befestigung der Fremdkörperbergeeinrichtung in der endoskopischen Vorrichtung.

Vorliegend sind der Befestigungsabschnitt und zumindest das Bergenetz des Bergeabschnitts gemeinsam einstückig ausgebildet, wobei diese einstückige Ausbildung dabei dadurch realisiert ist, dass zumindest das Bergenetz des Bergeabschnitts und der Befestigungsabschnitt durch ein textiles Gebilde realisiert sind. Dabei ist dieses textile Gebilde aus mindestens einem Filament hergestellt, welches verknüpft ist und hierdurch das textile Gebilde definiert. Insofern ist unter einem "textilen Gebilde" ein Flächen- oder Raumgebilde zu verstehen, welches durch das Verknüpfen eines oder mehrerer Filamente realisiert worden ist. Unter einem "Filament" ist im Sinne der Erfindung eine Faser mit bevorzugt praktisch unbegrenzter Länge zu verstehen, so dass es sich bei dem Filament dann also insbesondere um eine Endlosfaser handelt. Im Sinne der Erfindung umfasst der Begriff "Verknüpfen" dabei auch das Knüpfen des mindestens einen Filaments.

Im Rahmen der Erfindung könnte das textile Gebilde dabei dadurch gebildet sein, dass ein oder mehrere Filamente zu Maschen verschlungen sind. In diesem Fall besteht das textile Gebilde also aus Maschen, die jeweils durch Hineinschlingen eine mittels des mindestens einen Filaments gebildeten Schleife in eine andere durch das mindestens eine Filament gebildeten Schleife verwirklicht sind. Dementsprechend könnte das textile Gebilde bei dieser Variante der Erfindung auch als Maschenware bezeichnet werden. Die Maschen des textilen Gebildes könnten dabei durch Stricken eines einzigen Filaments oder mehrerer Filamente realisiert sein, so dass das textile Gebilde in diesem Fall als Strickware verwirklicht ist. Alternativ dazu könnte das textile Gebilde aber auch als textiles Gewirk hergestellt sein, beispielsweise als Kulierwirkware aus einem einzigen Filament oder als Kettenwirkware aus mehreren Filamenten. Hierdurch kann jeweils ein geeignetes textiles Gebilde zur Ausbildung des Befestigungsabschnitts und zumindest des Bergenetzes des Bergeabschnitts realisiert werden.

Alternativ könnte das textile Gebilde auch als Gewebe durch Weben mehrerer Filamente oder auch durch Flechten mehrerer Filamente realisiert sein. Denn auch hierdurch kann ein geeigneter Aufbau eines textilen Gebildes zur Realisierung des Befestigungsabschnitts und zumindest des Bergenetzes des Bergeabschnitts verwirklicht werden. Weiter alternativ könnte das textile Gebilde auch durch Knüpfen eines oder mehrerer Filamente gebildet sein, beispielsweise ähnlich Makramee, Platting oder Häkeln. Gemäß dem nebengeordneten Anspruch 1 umfasst die Erfindung nun die technische Lehre, dass das Gebilde bei dem Befestigungsabschnitt mindestens eine Montageöse bildet, an welcher die Befestigung der Fremdkörperbergeeinrichtung in der endoskopischen Vorrichtung herzustellen ist. Mit anderen Worten definiert das textile Gebilde an dem Befestigungsabschnitt der erfindungsgemäßen Fremdkörperbergeeinrichtung also mindestens eine Montageöse, an welcher die Befestigung der Fremdkörperbergeeinrichtung in der endoskopischen Vorrichtung erfolgt.

Eine derartige Ausgestaltung einer Fremdkörperbergeeinrichtung hat dabei den Vorteil, dass durch Bildung der mindestens einen Montageöse eine formschlüssige Befestigung an dem Befestigungsabschnitt der Fremdkörperbergeeinrichtung hergestellt werden kann, wodurch auch eine zuverlässige und belastbare Befestigung der erfindungsgemäßen Fremdkörperbergeeinrichtung in der endoskopischen Vorrichtung erfolgen kann. Aufgrund der einstückigen Ausführung zumindest des Bergernetzes und des Befestigungsabschnitts durch das textile Gebilde, welches dabei auch die mindestens eine Montageöse des Befestigungsabschnitts bildet, kann zudem der Herstellungsaufwand der erfindungsgemäßen Fremdkörperbergeeinrichtung niedrig gehalten werden. Denn bei der Herstellung kann die Anzahl an notwendigen Montageschritten reduziert werden, wobei das Verknüpfen des mindestens einen Filaments zudem auf einfache Art und Weise im Sinne eines kontinuierlichen Fertigungsprozesses automatisiert werden kann.

Darüber hinaus kann durch die zumindest teilweise Ausgestaltung der Fremdkörperbergeeinrichtung aus dem textilen Gebilde eine hohe Flexibilität der Fremdkörperbergeeinrichtung erreicht werden, wodurch diese leichter biegbar ist und damit auch bei Anwendung in einer endoskopischen Vorrichtung einfacher durch komplexe Wege navigiert werden kann. Hierbei wird zudem auch die Gefahr einer Verletzung des Patienten reduziert, wobei das mindestens eine Filament zusätzlich aus einem weichen Material hergestellt sein kann, um die Gefahr einer Verletzung weiter zu minimieren oder auch den Behandlungskomfort zu erhöhen. Bei geeigneter Wahl des Materials des mindestens einen Filaments kann hierdurch zudem eine Biokompatibilität erreicht und dadurch die Gefahr von Abstoßungsreaktionen oder Entzündungen reduziert und/oder das Gewicht der Fremdkörperbergeeinrichtung reduziert und/oder die Ergonomie bei Anwendung der Fremdkörperbergeeinrichtung erhöht werden.

Materialabhängig ist zudem eine zuverlässige Sterilisation möglich sowie eine hohe Lebensdauer der Fremdkörperbergeeinrichtung erreichbar. Außerdem lassen sich in Abhängigkeit des jeweiligen Materials des mindestens einen Filaments bestimmte Eigenschaften der Fremdkörperbergeeinrichtung realisieren, wie beispielsweise eine bestimmte Elastizität, eine bestimmte Steifigkeit und/oder eine bestimmte Leitfähigkeit, wobei diese Eigenschaften bei Kombination unterschiedlicher Materialien auch gezielt bereichsweise unterschiedlich gestaltet sein können.

Des Weiteren können über das textile Gebilde problemlos auch komplexe Gestaltungen der Fremdkörperbergeeinrichtung verwirklicht werden. Ferner können Sensoren oder Aktuatoren mit niedrigem Aufwand an geeigneter Stelle in das textile Gebilde eingebettet werden.

Im Sinne der Erfindung ist unter einer "Montageöse" ein an dem Befestigungsabschnitt ausgestalteter, ringähnlicher Bereich zu verstehen, durch welchen ein Durchgang umgrenzt ist. So kann das mindestens eine Filament die mindestens eine, ringähnliche Montageöse auch durch Überkreuzen in zumindest einem Bereich realisieren. Durch den umgrenzten Durchgang kann dabei ein seitens der restlichen endoskopischen Vorrichtung vorgesehenes Befestigungselement, beispielsweise ein Haken oder ähnliches, hindurchgeführt werden, um eine formschlüssige Verbindung zwischen dem Befestigungsabschnitt der Fremdkörperbergeeinrichtung und der restlichen endoskopischen Vorrichtung herzustellen.

Entsprechend einer Ausführungsform der Erfindung ist das Bergenetz bei dem Bergeabschnitt von einem Tragrahmen umgeben. Dabei bildet dieser Tragrahmen insbesondere eine tragende Struktur des Bergeabschnitts, wobei dieser Tragrahmen dabei bevorzugt einer Umrandung des Bergenetzes dient. Weiter bevorzugt wird über den Tragrahmen dann ein Aufspannen des Bergenetzes realisiert, wenn die erfindungsgemäße Fremdkörperbergeeinrichtung im verbauten Zustand in der endoskopischen Vorrichtung mit ihrem Bergeabschnitt insbesondere aus einem Tubus der endoskopischen Vorrichtung ausgeschoben wird, um im aufgespannten Zustand des Bergenetzes ein zu bergendes Objekt aufnehmen zu können. Bevorzugt ist der Tragrahmen vollständig oder nahezu vollständig umlaufend zu dem Bergenetz ausgestaltet, wobei der Tragrahmen im Rahmen der Erfindung drahtähnlich aus einem Element oder auch mehreren Elementen gebildet sein kann. So kann der Tragrahmen als Drahtschlaufe vorliegen.

Besonders bevorzugt ist der Tragrahmen aber gemeinsam mit dem Bergenetz und dem Befestigungsabschnitts einstückig als das textile Gebilde ausgestaltet und weist im Vergleich zu dem Bergenetz eine höhere Steifigkeit auf. Hierdurch kann der Herstellungsaufwand der erfindungsgemäßen Fremdkörperbergeeinrichtung weiter reduziert werden, indem neben dem Bergenetz an dem Befestigungsabschnitt auch der Tragrahmen des Bergeabschnitts durch das textile Gebilde ausgestaltet wird. Gleichzeitig kann aufgrund der im Vergleich zum Bergenetz höheren Steifigkeit des Tragrahmens eine geeignete Struktur zum Tragen des Bergenetzes sowie auch zu dessen Aufspannen verwirklicht werden.

In Weiterbildung der vorgenannten Variante ist die höhere Steifigkeit des Tragrahmens im Vergleich zu dem Bergenetz verwirklicht, indem der Tragrahmen im Vergleich zu dem Bergenetz mit einer höheren Dichte gebildet ist. Die höhere Steifigkeit kann im Rahmen der Erfindung dadurch erreicht sein, dass das textile Gebilde den Tragrahmen im Vergleich zu dem Bergenetz mit einer höheren Dichte, d.h. einer höheren Konzentration an Filament realisiert. Bei Ausführung des textilen Gebildes als Strickware könnte der Tragrahmen dabei im Krausstrickmuster realisiert sein, während das Bergenetz im Glattstrickmuster gefertigt ist. Alternativ oder ergänzend dazu kann die höhere Steifigkeit aber auch verwirklicht sein, indem das textile Gebilde aus mehreren Filamenten gebildet und dabei bei der Herstellung des textilen Gebildes an dem Tragrahmen im Vergleich zu der Herstellung des textilen Gebildes an dem Bergenetz Filament mit einer höheren Dichte verwendet worden ist. Weiter alternativ oder ergänzend könnte die höhere Steifigkeit des Tragrahmens zudem auch dadurch realisiert sein, dass das textile Gebilde aus mehreren Filamenten gebildet und dabei zur Gestaltung des Tragrahmens ein Filament aus einem Material mit einer höheren Festigkeit verwendet worden ist.

Weiter bevorzugt bildet der Tragrahmen auch einen Übergang zu dem Befestigungsabschnitt. In vorteilhafter Weise wird hierdurch ein geeigneter, belastbarer Übergang des Bergeabschnitts in den hieran angrenzenden Befestigungsabschnitt realisiert.

Gemäß einer Ausgestaltungsmöglichkeit der Erfindung weist der Befestigungsabschnitt im Vergleich zu dem Bergenetz eine höhere Steifigkeit auf. Dadurch kann bei der erfindungsgemäßen Fremdkörperbergeeinrichtung ein belastbarer Befestigungsabschnitt realisiert werden, über welchen zudem lineare Stellbewegungen auf den Bergeabschnitt übertragbar sind.

Bevorzugt ist die höhere Steifigkeit des Befestigungsabschnitts im Vergleich zu dem Bergenetz verwirklicht, indem der Befestigungsabschnitt im Vergleich zu dem Bergenetz mit einer höheren Dichte gebildet ist. Das textile Gebilde kann den Befestigungsabschnitt dabei mit einer höheren Dichte bilden, d.h. einer höheren Konzentration an Filament. Bei Ausführung des textilen Gebildes als Strickware ist der Befestigungsabschnitt dann bevorzugt im Krausstrickmuster realisiert, während das Bergenetz insbesondere im Glattstrickmuster gefertigt ist. Alternativ oder ergänzend dazu könnte aber die höhere Steifigkeit des Befestigungsabschnitts auch dadurch verwirklicht sein, dass das textile Gebilde aus mehreren Filamenten gebildet ist und bei der Herstellung des textilen Gebildes an dem Befestigungsabschnitt im Vergleich zu der Herstellung des textilen Gebildes an dem Bergenetz Filament mit einer höheren Dichte verwendet worden ist. Weiter alternativ oder ergänzend kann bei einer Herstellung des textilen Gebildes aus mehreren Filamenten zur Gestaltung des Befestigungsabschnitts auch ein Filament aus einem Material mit einer höheren Festigkeit zur Anwendung gekommen sein.

Es ist eine weitere Ausführungsform der Erfindung, dass der Befestigungsabschnitt an den Bergeabschnitt mit einem länglichen Verbindungsteil angebunden ist, welcher den Bergeabschnitt mit der mindestens einen Montageöse verbindet. Hierdurch kann die Befestigung der Fremdkörperbergeeinrichtung problemlos beabstandet zu dem Bergeabschnitt und dem hier ausgebildeten Bergenetz erfolgen. In Weiterbildung dieser Ausführungsform kann der Verbindungsteil zudem ein Betätigungselement der endoskopischen Vorrichtung bilden, wobei die mindestens eine Montageöse für eine Befestigung an einem Handteil der endoskopischen Vorrichtung vorgesehen ist. In diesem Fall wäre der längliche Verbindungsteil dann mit einer derartigen Erstreckung auszuführen, dass über den Verbindungsteil direkt die Verbindung zu dem Handteil der endoskopischen Vorrichtung vorgenommen werden und in der Folge ein zwischenliegendes, separates Betätigungselement, wie beispielsweise ein Betätigungsdraht oder -seil entfallen kann.

In Weiterbildung der Erfindung ist das mindestens eine Filament als Monokomponente ausgeführt, besteht also nur aus einem Material. Bevorzugt ist das mindestens eine Filament dabei aus einem thermoplastischen Kunststoff gebildet, wobei es sich dabei besonders bevorzugt um Polyvinylchlorid oder Polyethylen handelt.

Entsprechend einer Variante der Erfindung bildet das textile Gebilde bei dem Befestigungsabschnitt genau eine Montageöse. Hierdurch kann eine Befestigung der erfindungsgemäßen Fremdkörperbergeeinrichtung auf kompaktem Raum verwirklicht werden.

Alternativ dazu bildet das textile Gebilde bei dem Befestigungsabschnitt mehrere Montageösen, wobei hierbei bevorzugt genau zwei Montageösen oder genau drei Montageösen gebildet sind. Ganz besonders bevorzugt folgen die mehreren Montageösen dabei in Reihe aufeinander, d.h. die Montageösen sind durch das textile Gebilde in Reihe aufeinanderfolgend gebildet. Die Bildung mehrerer Montageösen hat dabei den Vorteil, dass die Befestigung der erfindungsgemäßen Fremdkörperbergeeinrichtung in der endoskopischen Vorrichtung durch Durchfädeln eines Zugelements der endoskopischen Vorrichtung stattfinden kann. Dadurch ist dann kein Knicken des Zugelements notwendig. Ferner kann hierdurch die Sicherheit der Montage erhöht und die Stabilität der Befestigung verbessert werden, wobei hierbei auch ein Verpressen der Verbindung vereinfacht ist.

Gegenstand der Erfindung ist zudem eine endoskopische Vorrichtung, welche einen flexiblen Tubus umfasst. Dieser Tubus weist dabei zumindest ein in seiner Längsrichtung verlaufendes Lumen auf, durch welches ein Betätigungselement mit radialem Spiel verläuft. An diesem Betätigungselement ist dabei eine Fremdkörperbergeeinrichtung nach einem oder mehreren der vorstehend beschriebenen Varianten angebunden. Die Verwendung der erfindungsgemäßen Fremdkörperbergeeinrichtung in der endoskopischen Vorrichtung hat dabei den Vorteil, dass sich die endoskopische Vorrichtung damit mit niedrigem Herstellungsaufwand und unter zuverlässiger Befestigung der Fremdkörperbergeeinrichtung realisieren lässt.

Die Fremdkörperbergeeinrichtung kann über das Betätigungselement bevorzugt zwischen einer vollständig in den Tubus eingezogenen Position und einer Position bewegt werden, in welcher zumindest der Bergeabschnitt der Fremdkörperbergeeinrichtung überwiegend aus dem Tubus ausgeschoben ist. In der eingefahrenen Position ist das Bergenetz der Fremdkörperbergeeinrichtung dabei insbesondere in dem Tubus zusammengefaltet, während es in der ausgefahrenen Position bevorzugt aufgespannt ist.

Besonders bevorzugt ist die erfindungsgemäße Fremdkörperbergeeinrichtung an der Montageöse des Befestigungsabschnitts an dem Betätigungselement befestigt, wobei das unter Spiel im Lumen geführte Betätigungselement hierbei insbesondere als Betätigungsdraht oder -seil vorliegt.

Alternativ dazu ist es aber auch eine Ausgestaltungsmöglichkeit der Erfindung, dass das Betätigungselement durch den Verbindungsteil der Fremdkörperbergeeinrichtung gebildet und die Montageöse der Fremdkörperbergeeinrichtung an einem Handteil befestigt ist. In diesem Fall ist der Befestigungsabschnitt der Fremdkörperbergeeinrichtung also mit dem länglichen Verbindungsteil ausgestattet, welcher dann innerhalb der endoskopischen Vorrichtung als Betätigungselement fungiert und sich dazu ausgehend von dem Bergeabschnitt bis zu dem Handteil der endoskopischen Vorrichtung erstreckt, um seitens des Handteils die Befestigung über die Montageöse des Befestigungsabschnitts vorzunehmen. In vorteilhafter Weise kann hierdurch ein separates Betätigungselement entfallen, indem der Befestigungsabschnitt der erfindungsgemäßen Fremdkörperbergeeinrichtung zusätzlich das Betätigungselement bildet.

Gemäß dem nebengeordneten Anspruch 12 werden bei einem Verfahren zur Herstellung einer Fremdkörperbergeeinrichtung ein ein Bergenetz bildender Bergeabschnitt und ein hieran angrenzender und für eine Befestigung der Fremdkörperbergeeinrichtung in der endoskopischen Vorrichtung vorgesehener Befestigungsabschnitt hergestellt, wobei dabei zumindest das Bergenetz des Bergeabschnitts und der Befestigungsabschnitt gemeinsam einstückig als textiles Gebilde ausgestaltet werden, welches durch Verknüpfen mindestens eines Filaments gebildet wird.

Im Rahmen der erfindungsgemäßen Herstellung einer Fremdkörperbergeeinrichtung werden also ein Bergeabschnitt und ein Befestigungsabschnitt angrenzend zueinander ausgestaltet, wobei der Bergeabschnitt und der Befestigungsabschnitt dabei besonders bevorzugt unmittelbar benachbart zueinander ausgebildet werden. Bei dem Bergeabschnitt wird dabei zudem ein Bergenetz realisiert, welches für die Aufnahme eines zu entfernenden Fremdkörpers, wie beispielsweise eines Polypen, vorgesehen ist.

Bei der erfindungsgemäßen Herstellung werden der Befestigungsabschnitt und zumindest das Bergenetz des Bergeabschnitts gemeinsam einstückig ausgebildet, wobei der Befestigungsabschnitt und zumindest das Bergenetz des Bergeabschnitts dazu durch ein textiles Gebilde definiert werden. Dieses textile Gebilde wird aus mindestens einem Filament hergestellt, welches zur Ausgestaltung des textilen Gebildes verknüpft wird. Dementsprechend ist unter einem "textilen Gebilde" ein Flächen- oder Raumgebilde zu verstehen, welches durch das Verknüpfen eines oder mehrerer Filamente realisiert worden ist. Unter einem "Filament" ist im Sinne der Erfindung eine Faser mit bevorzugt praktisch unbegrenzter Länge zu verstehen, so dass es sich bei dem Filament dann also insbesondere um eine Endlosfaser handelt. Im Sinne der Erfindung umfasst der Begriff "Verknüpfen" dabei auch das Knüpfen des mindestens einen Filaments.

Konkret könnte das textile Gebilde dabei dadurch gebildet werden, dass ein oder mehrere Filamente zu Maschen verschlungen werden. In diesem Fall wird das textile Gebilde also aus Maschen gebildet, die jeweils durch Hineinschlingen eine mittels des mindestens einen Filaments gebildeten Schleife in eine andere durch das mindestens eine Filament gebildeten Schleife verwirklicht werden. Dementsprechend könnte das sich bildende, textile Gebilde bei dieser Variante der Erfindung auch als Maschenware bezeichnet werden. Die Maschen des textilen Gebildes könnten dabei durch Stricken eines einzigen Filaments oder mehrerer Filamente realisiert werden, so dass das textile Gebilde in diesem Fall als Strickware realisiert wird. Alternativ dazu könnte das textile Gebilde aber auch als textiles Gewirk hergestellt werden, beispielsweise als Kulierwirkware aus einem einzigen Filament oder als Kettenwirkware aus mehreren Filamenten. Auch hierdurch wird jeweils ein geeignetes, textiles Gebilde zur Ausbildung des Befestigungsabschnitts und zumindest des Bergenetzes des Bergeabschnitts realisiert. Alternativ könnte das textile Gebilde auch als Gewebe durch Weben mehrerer Filamente oder auch durch Flechten mehrerer Filamente hergestellt werden. Denn auch hierdurch wird ein geeigneter Aufbau eines textilen Gebildes zur Realisierung des Befestigungsabschnitts und zumindest des Bergenetzes des Bergeabschnitts verwirklicht. Weiter alternativ könnte das textile Gebilde auch durch Knüpfen eines oder mehrerer Filamente gebildet sein, beispielsweise ähnlich Makramee, Platting oder Häkeln.

Gemäß dem kennzeichnenden Teil des nebengeordneten Anspruchs 12 umfasst die Erfindung nun die technische Lehre, dass durch das Gebilde bei dem Befestigungsabschnitt mindestens eine Montageöse gebildet wird, an welcher die Befestigung der Fremdkörperbergeeinrichtung in der endoskopischen Vorrichtung herzustellen ist. Mit anderen Worten wird also bei der Herstellung des textilen Gebildes durch das Gebilde mindestens eine Montageöse definiert, wobei diese mindestens eine Montageöse der Befestigung der Fremdkörperbergeeinrichtung in der endoskopischen Vorrichtung dient.

Die erfindungsgemäße Herstellung einer Fremdkörperbergeeinrichtung hat dabei den Vorteil, dass hierdurch die Herstellung hierdurch mit niedrigem Aufwand möglich ist, indem zumindest das Bergenetz des Bergeabschnitts und der Befestigungsabschnitt durch Verknüpfen des mindestens einen Filaments im Zuge der Herstellung des textilen Gebildes definiert werden. Denn bei der Herstellung kann die Anzahl an notwendigen Montageschritten reduziert werden, wobei das Verknüpfen des mindestens einen Filaments zudem auf einfache Art und Weise automatisierbar ist. Darüber hinaus können problemlos Anpassungen an unterschiedliche, zu fertigende Abmessungen der Fremdkörperbergeeinrichtung realisiert werden, ohne dass hierfür Maschinenseitig weitreichende Veränderungen vorzunehmen sind.

In Weiterbildung der Erfindung wird die Fremdkörperbergeeinrichtung im Rahmen des erfindungsgemäßen Verfahrens im Weiteren nach einem oder mehreren der vorstehend beschriebenen Varianten ausgestaltet. Hierdurch ist jeweils ein geeigneter Aufbau der Fremdkörperbergeeinrichtung realisierbar.

Alternativ oder ergänzend dazu werden im Rahmen des erfindungsgemäßen Verfahrens mehrere derartige Fremdkörperbergeeinrichtungen aufeinanderfolgend einstückig als textiles Gebilde durch Verknüpfen mindestens eines Filaments hergestellt, wobei die Fremdkörperbergeeinrichtungen anschließend an Trennstellen voneinander separiert werden. Hierdurch kann der Herstellungsaufwand weiter reduziert werden, indem eine fortlaufende Herstellung von Fremdkörperbergeeinrichtungen durchgeführt wird. Dabei können Fremdkörperbergeeinrichtungen in Reihe aufeinanderfolgend gefertigt werden, indem auf die Bildung eines Befestigungsabschnitts der vorhergehenden Fremdkörperbergeeinrichtung mittels des textilen Gebildes der Bergeabschnitt der nachfolgenden Fremdkörperbergeeinrichtung oder umgekehrt definiert wird. Alternativ oder auch ergänzend dazu können Fremdkörperbergeeinrichtungen zudem auch nebeneinanderliegend gefertigt werden, beispielsweise indem das textile Gebilde angrenzend an den Bergeabschnitt der einen Fremdkörperbergeeinrichtung direkt den Bergeabschnitt einer benachbarten Fremdkörperbergeeinrichtung bildet. Dadurch kann die Herstellung problemlos fortlaufend realisiert werden. Im Anschluss an eine gemeinsame Herstellung mehrerer Fremdkörperbergeeinrichtungen wird dann eine Auftrennung in die einzelnen Fremdkörperbergeeinrichtungen durch Separierung an den entsprechenden Trennstellen vorgenommen.

In Weiterbildung der vorgenannten Ausführungsform wird die Separierung mittels Heißschneiden vorgenommen. Hierdurch kann zum einen eine einfache Trennung realisiert sowie im Falle der Verwendung eines thermoplastischen Materials als Filament gleichzeitig ein ungewolltes Aufgehen des textilen Gebildes durch Verschmelzen des Materials unterbunden werden. Alternativ können die Trennstellen der Fremdkörperbergeeinrichtungen auch als Sollbruchstellen gestaltet sein, so dass die Separierung der Fremdkörperbergeeinrichtungen dann durch Umbiegen an den Sollbruchstellen stattfinden kann.

Vorteilhafte Ausführungsformen der Erfindung, die nachfolgend erläutert werden, sind in den Zeichnungen dargestellt. Es zeigt:
- Fig. 1: eine Ansicht einer Fremdkörperbergeeinrichtung gemäß einer ersten Ausführungsform der Erfindung;
- Fig. 2: eine perspektivische Ansicht der Fremdkörperbergeeinrichtung aus Fig. 1 ;
- Fig. 3: eine Schnittansicht eines Teils einer endoskopischen Vorrichtung mit der Fremdkörperbergeeinrichtung nach den Fig. 1 und 2;
- Fig. 4 und 5: alternative Ausgestaltungen eines Befestigungselements einer endoskopischen Vorrichtung zur Befestigung der Fremdkörperbergeeinrichtung aus den Fig. 1 und 2;
- Fig. 6: einen Fertigungsschritt einer aufeinanderfolgenden Herstellung erfindungsgemäßer Fremdkörperbergeeinrichtungen nach den Fig. 1 und 2, gezeigt vor einer Separierung in die einzelnen Fremdkörperbergeeinrichtungen;
- Fig. 7: eine Ansicht eines Befestigungselements einer endoskopischen Vorrichtung und einer Fremdkörperbergeeinrichtung entsprechend einer zweiten Ausgestaltungsmöglichkeit der Erfindung, gezeigt vor einer Befestigung der Fremdkörperbergeeinrichtung;
- Fig. 8: eine weitere Ansicht des Befestigungselements und eines Befestigungsabschnitts der Fremdkörperbergeeinrichtung aus Fig. 7, gezeigt im Zuge der Befestigung der Fremdkörperbergeeinrichtung; und
- Fig. 9: eine Ansicht eines Befestigungselements einer endoskopischen Vorrichtung und einen Befestigungsabschnitt einer Fremdkörperbergeeinrichtung gemäß einer dritten Ausführungsform der Erfindung, gezeigt nach einer Befestigung der Fremdkörperbergeeinrichtung.

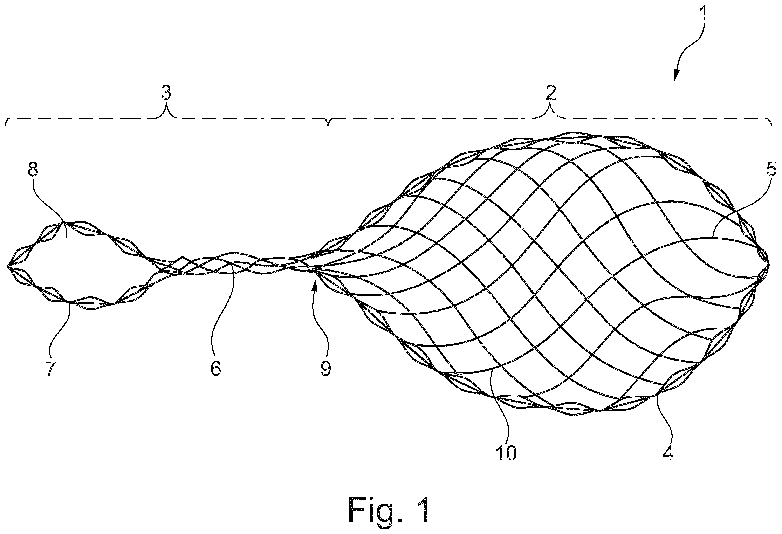

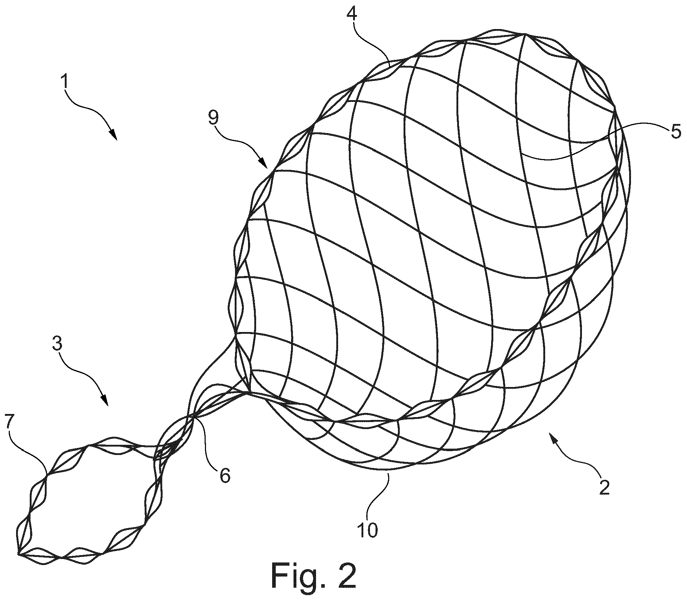

Aus den Fig. 1 und 2 gehen Ansichten einer Fremdkörperbergeeinrichtung 1 hervor, welche entsprechend einer ersten Ausführungsform der Erfindung ausgestaltet ist. Die Fremdkörperbergeeinrichtung 1 umfasst dabei einen Bergeabschnitt 2 und einen Befestigungsabschnitt 3, die aneinander angrenzen, so dass der Befestigungsabschnitt 3 also unmittelbar benachbart zu dem Bergeabschnitt 2 ausgestaltet ist.

Der Bergeabschnitt 2 weist einen Tragrahmen 4 auf, durch welchen ein Bergenetz 5 des Bergeabschnitts 2 umgrenzt ist. Wie in Fig. 2 zu erkennen ist, in welcher die Fremdkörperbergeeinrichtung 1 in einem entfalteten Zustand des Bergenetzes 5 gezeigt ist, ist das Bergenetz 5 dabei in seinem entfalteten Zustand bauchig gestaltet.

Wie in den Fig. 1 und 2 jeweils zu erkennen ist, bildet der Tragrahmen 4 des Bergeabschnitts 2 zudem einen Übergang zum Befestigungsabschnitt 3, welcher dabei an den Tragrahmen 4 mit einem länglich gestalteten Verbindungsteil 6 anknüpft. Dabei verbindet der Verbindungsteil 6 den Tragrahmen 4 mit einer Montageöse 7 des Befestigungsabschnitts 3, wobei diese Montageöse 7 hierbei ringförmig gestaltet ist und einen Durchgang 8 definiert, an welchem eine formschlüssige Befestigung der Fremdkörperbergeeinrichtung 1 erfolgen kann.

Vorliegend ist die Fremdkörperbergeeinrichtung 1 einstückig als textiles Gebilde 9 ausgestaltet, welches durch Verknüpfen eines Filaments 10 hergestellt worden ist. Das Filament ist dabei als Monokomponente ausgeführt und besteht insbesondere aus einem thermoplastischen Kunststoff, bei welchem es sich um Polyvinylchlorid oder Polyethylen handeln kann. Das textile Gebilde 9 ist dabei durch Verstricken des Filaments 10 ausgestaltet, indem das Filament 10 zu Maschen verschlungen und das Gebilde 9 insofern als Strickware gestaltet ist. Hierbei bildet das textile Gebilde 9 den Bergeabschnitt 2 und den Befestigungsabschnitt 3 einstückig aus, so dass in der Folge auch der Tragrahmen 4, das Bergenetz 5, der Verbindungsteil 6 und die Montageöse 7 einteilig durch das textile Gebilde 9 realisiert sind.

Wie insbesondere in Fig. 2 angedeutet ist, ist das Gebilde 9 in den den Tragrahmen 4, den Verbindungsteil 6 und die Montageöse 7 ausbildenden Bereichen mit einer höheren Dichte der Maschen ausgebildet, als in dem das Bergenetz 5 bildenden Bereich. Hierdurch weist die Fremdkörperbergeeinrichtung 1 an dem Tragrahmen 4, dem Verbindungsteil 6 und der Montageöse 7 im Vergleich zum Bergenetz 5 jeweils eine höhere Steifigkeit auf. Bei dem Tragrahmen 4 wird hierdurch ein zuverlässiges Tragen des Bergenetzes 5 sowie ein Aufspannen des Bergenetzes 5 realisiert, wohingegen über den Verbindungsteil 6 aufgrund der höheren Steifigkeit eine lineare Kraftübertragung zwischen der Montageöse 7 und dem Bergeabschnitt 2 darstellbar ist. Die Montageöse 7 ermöglicht aufgrund der höheren Steifigkeit eine belastbare Befestigung der Fremdkörperbergeeinrichtung 1. Die höhere Dichte der Maschen des Gebildes 9 ist dabei im Bereich des Tragrahmens 4, des Verbindungsteils 6 und der Montageöse 7 insbesondere dadurch realisiert, dass das Verstricken des Filaments 10 hier jeweils im Krausstrickmuster vorgenommen ist, wohingegen das Verstricken des Filaments 10 zur Ausgestaltung des Bergenetzes 5 bevorzugt im Glattstrickmuster vollzogen ist.

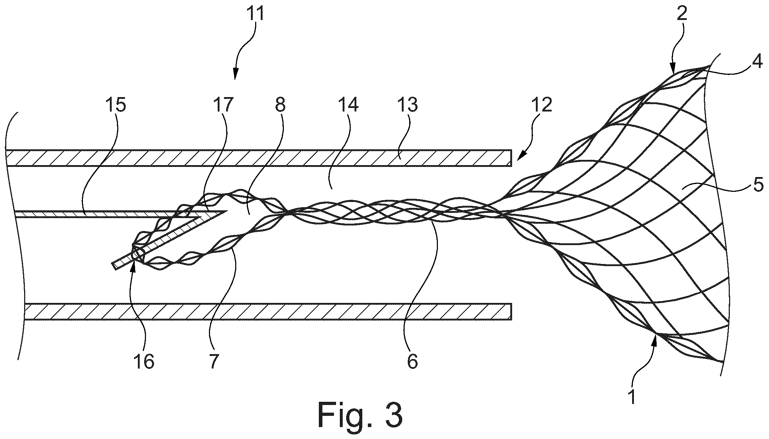

Fig. 3 zeigt eine Schnittansicht eines Teils einer endoskopischen Vorrichtung 11, wobei diese endoskopische Vorrichtung 11 dabei im Bereich eines Endes 12 eines flexiblen Tubus 13 gezeigt ist. Dieser Tubus 13 weist ein in seiner Längsrichtung verlaufendes Lumen 14 auf, in welchem ein Betätigungselement 15 mit radialem Spiel verläuft. Bei dem Betätigungselement 15 handelt es sich vorliegend um einen Betätigungsdraht oder -seil, wobei das Betätigungselement 15 an seinem in Fig. 3 zu sehenden Ende ein Befestigungselement 16 bildet, an welchem die erfindungsgemäße Fremdkörperbergeeinrichtung 1 mit ihrer Montageöse 7 befestigt ist. Das Befestigungselement 16 ist dabei als widerhakenartig gestalteter Befestigungshaken 17 ausgestaltet, welcher in den durch die Montageöse 7 gebildeten Durchgang 8 der Fremdkörperbergeeinrichtung 1 eingeführt ist. Hierdurch wird eine formschlüssige Befestigung der Fremdkörperbergeeinrichtung 1 an dem Betätigungselement 15 realisiert.

An einem zu der Befestigung der Fremdkörperbergeeinrichtung 1 entgegengesetzt liegenden Ende ist das Betätigungselement 15 zudem auf dem Fachmann prinzipiell bekannte Weise mit einem - vorliegend nicht weiter dargestellten - Handteil verbunden, über welchen eine lineare Stellbewegung in das Betätigungselement 15 eingeleitet werden kann. Diese lineare Stellbewegung wird über das Betätigungselement 15 dann aufgrund der formschlüssigen Verbindung auch auf die Fremdkörperbergeeinrichtung 1 übertragen. Hierdurch kann die Fremdkörperbergeeinrichtung 1 zwischen einem vollständig in den Tubus 13 eingezogenen Zustand und einem, in Fig. 3 angedeuteten Zustand verschoben werden, in welchem die Fremdkörperbergeeinrichtung 1 mit ihrem Bergeabschnitt 2 aus dem Tubus 13 ausgeschoben ist. In dem eingezogenen Zustand ist der Tragrahmen 4 des Bergeabschnitts 2 dabei zusammengelegt und dementsprechend das Bergenetz 5 zusammengeklappt, wohingegen der Tragrahmen 4 in dem ausgeschobenen Zustand für das in Fig. 2 gezeigte Aufklappen des Bergenetzes 5 sorgt. Hierdurch können über die Fremdkörperbergeeinrichtung in dem ausgeschobenen Zustand Fremdkörper, beispielsweise Polypen, mit dem Bergenetz 5 aufgenommen werden. Aufgrund der höheren Steifigkeit des Verbindungsteils 6 wird dabei eine zuverlässige Übertragung der linearen Stellbewegung von dem Betätigungselement 15 auf den Bergeabschnitt 2 der Fremdkörperbergeeinrichtung 1 realisiert.

Im Rahmen der Erfindung wäre jedoch auch eine Variante denkbar, bei welcher eine erfindungsgemäß ausgestaltete Fremdkörperbergeeinrichtung unmittelbar an einem Handteil der endoskopischen Vorrichtung befestigt ist. Dazu müsste dann der Verbindungsteil des Befestigungsabschnitts dieser Fremdkörperbergeeinrichtung mit einer entsprechenden Länge und auch Steifigkeit ausgestaltet sein.

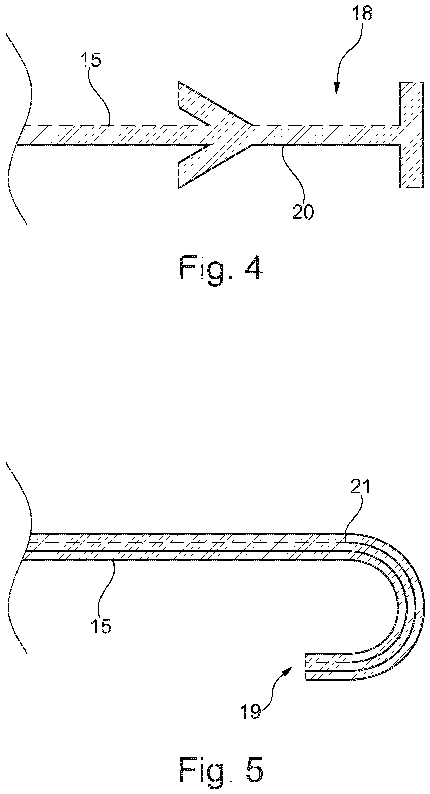

Ferner gehen aus den Fig. 4 und 5 alternative Gestaltungen von Befestigungselementen 18 bzw. 19 des Betätigungselements 15 hervor. Dabei sind auch die Befestigungselemente 18 und 19 als Befestigungshaken 20 und 21 gestaltet, die hierbei jedoch abweichend zu dem Befestigungshaken 17 aus Fig. 3 ausgebildet sind.

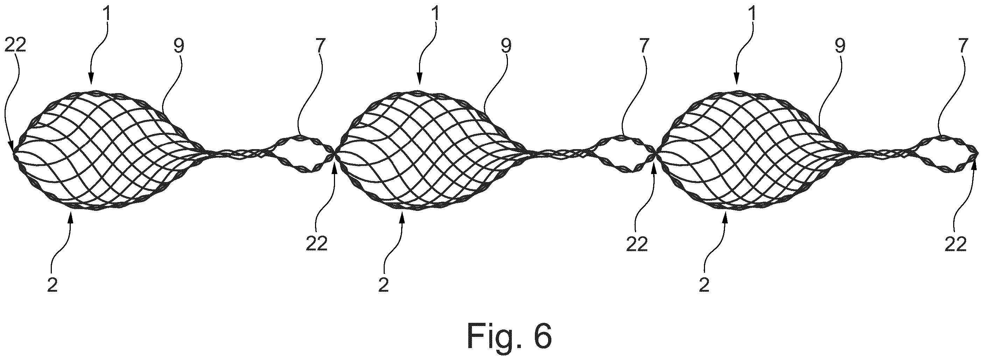

Fig. 6 zeigt einen Fertigungsabschnitt einer Herstellung der erfindungsgemäßen Fremdkörperbergeeinrichtung 1. Dabei werden im Rahmen dieser Herstellung mehrere Fremdkörperbergeeinrichtungen 1 einstückig und in Reihe aufeinanderfolgend durch Verstricken des Filaments 10 gefertigt, indem beim Verstricken des Filaments 10 zu der Montageöse 7 der einen Fremdkörperbergeeinrichtung 1 das Filament 10 im Folgenden dann zur Ausbildung des Bergeabschnitts 2 der in der Reihe nachfolgenden Fremdkörperbergeeinrichtung 1 verstrickt wird. Anschließend werden die in Reihe aufeinanderfolgenden Fremdkörperbergeeinrichtungen 1 dann separiert, indem die Reihe der Fremdkörperbergeeinrichtungen 1 an Trennstellen 22 jeweils aufgetrennt wird. Dieses Auftrennen erfolgt dabei durch Heißschneiden, wodurch das Filament 10 an den Trennstellen 22 verschmolzen und dadurch ein ungewolltes Aufgehen des jeweiligen Gebildes 9 der einzelnen Fremdkörperbergeeinrichtung 1 verhindert wird. Alternativ dazu könnten die Trennstellen 22 auch als Sollbruchstellen gestaltet sein, so dass eine Separierung der Fremdkörperbergeeinrichtungen 1 dann durch Umbiegen an den Trennstellen 22 herbeigeführt werden könnte.

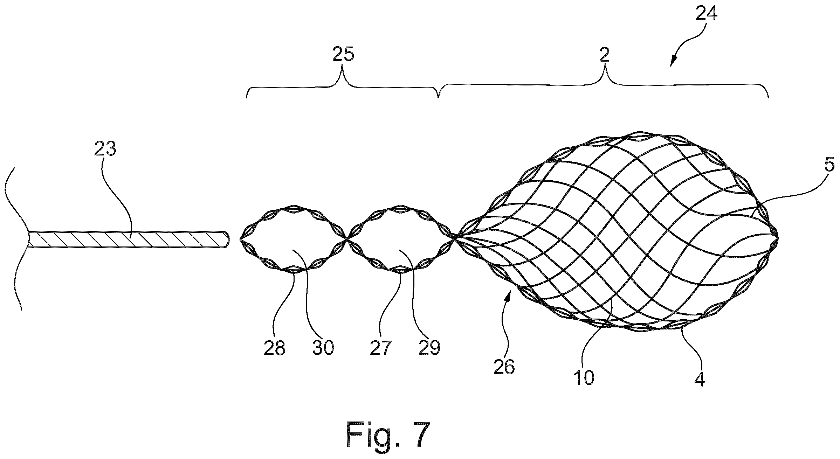

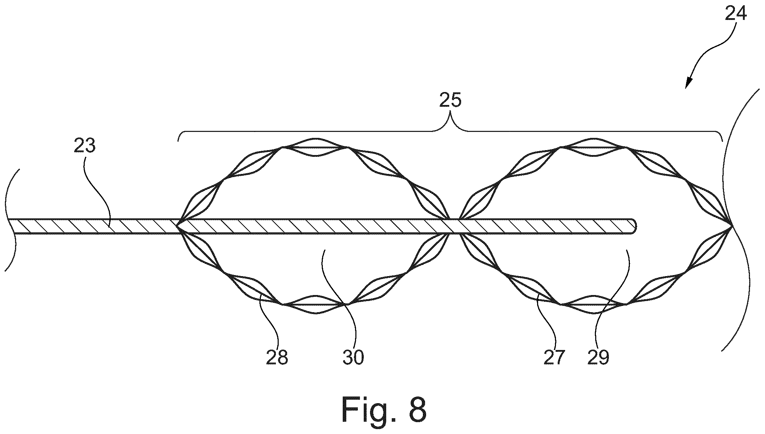

Des Weiteren gehen aus den Fig. 7 und 8 Ansichten eines Betätigungselements 23 einer - in den Fig. 7 und 8 jeweils nicht weiter gezeigten - endoskopischen Vorrichtung und einer Fremdkörperbergeeinrichtung 24 hervor. Dabei ist die Fremdkörperbergeeinrichtung 24 entsprechend einer zweiten Ausgestaltungsmöglichkeit der Erfindung ausgebildet und entspricht weitestgehend der Fremdkörperbergeeinrichtung 1 aus den Fig. 1 und 2. So umfasst auch die Fremdkörperbergeeinrichtung 24 einen Bergeabschnitt 2 und einen hieran angrenzenden Befestigungsabschnitt 25, welche gemeinsam einstückig als textiles Gebilde 26 ausgestaltet sind, das durch Verknüpfen des Filaments 10 hergestellt worden ist.

Während der Bergeabschnitt 2 der Fremdkörperbergeeinrichtung 24 zumindest im Wesentlichen identisch zu dem Bergeabschnitt 2 der Fremdkörperbergeeinrichtung 1 aus den Fig. 1 und 2 gestaltet ist, weist der Befestigungsabschnitt 25 gegenüber der Variante aus den Fig. 1 und 2 eine abweichende Gestaltung auf. So sind bei dem Befestigungsabschnitt 25 zwei Montageösen 27 und 28 gebildet, welche in Reihe aufeinanderfolgend jeweils ringförmig gestaltet sind und jeweils je einen Durchgang 29 bzw. 30 definieren. Hierbei liegt die Montageöse 27 zwischen der Montageöse 28 und dem angrenzenden Bergeabschnitt 2. Analog zu der Variante nach den Fig. 1 und 2 ist das Gebilde 26 in den den Tragrahmen 4 und die beiden Montageösen 27 und 28 ausbildenden Bereichen mit einer höheren Dichte der Maschen ausgebildet, als in dem das Bergenetz 5 bildenden Bereich, wodurch der Tragrahmen 4 und auch die beiden Montageösen 27 und 28 im Vergleich zum Bergenetz 5 des Bergeabschnitts 2 jeweils eine höhere Steifigkeit aufweisen. Auch im Übrigen entspricht die Fremdkörperbergeeinrichtung 24 sonst der Variante nach den Fig. 1 und 2, so dass hinsichtlich der weiteren Gestaltung auf das zu Fig. 1 und 2 beschriebene Bezug genommen wird.

Das Betätigungselement 23, bei welchem es sich um einen Betätigungsdraht oder -seil handeln kann, ist an seinem in den Fig. 7 und 8 zu sehenden Ende nicht mit einem Befestigungselement ausgestattet. Stattdessen wird das Betätigungselement 23 für die Befestigung an der Fremdkörperbergeeinrichtung 24 durch die Durchgänge 29 und 30 der Montageösen 27 und 28 hindurchgefädelt, wie in Fig. 8 angedeutet ist. Anschließend wird die Befestigung dann durch Verpressen des Betätigungselements 23 mit dem Befestigungsabschnitt 25 realisiert.

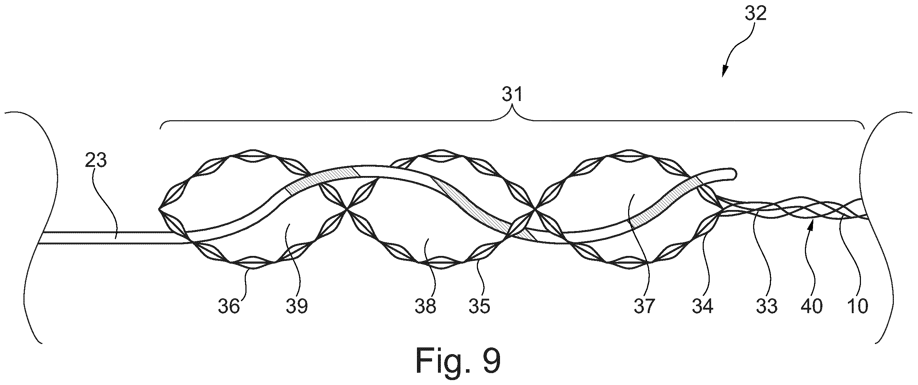

Schließlich zeigt noch Fig. 9 eine Ansicht des Betätigungselements 23 im Bereich einer Befestigung an einem Befestigungsabschnitt 31 einer Fremdkörperbergeeinrichtung 32, die hierbei entsprechend einer dritten Ausführungsform ausgestaltet und von welcher in Fig. 9 lediglich der Befestigungsabschnitt 31 gezeigt ist. Die Fremdkörperbergeeinrichtung 32 entspricht hierbei erneut weitestgehend der Fremdkörperbergeeinrichtung nach den Fig. 1 und 2, indem die Fremdkörperbergeeinrichtung 32 neben dem Befestigungsabschnitt 31 über einen - in Fig. 9 nicht gezeigten - Bergeabschnitt verfügt, welcher zumindest im Wesentlichen dem Bergeabschnitt 2 der Fremdkörperbergeeinrichtung 1 aus den Fig. 1 und 2 entspricht.

Im Unterschied zu der Fremdkörperbergeeinrichtung 1 sind bei dem Befestigungsabschnitt 31 der Fremdkörperbergeeinrichtung 32 neben einem Verbindungsteil 33, über welchen die Verbindung zu dem angrenzenden Bergeabschnitt hergestellt ist, aufeinanderfolgend drei Montageösen 34, 35 und 36 definiert, die jeweils ringförmig gestaltet sind und dadurch je einen Durchgang 37 bzw. 38 bzw. 39 bilden. Die Montageösen 34 bis 36 folgen dabei in Reihe aufeinander, indem die Montageöse 34 angrenzend zu dem Verbindungsteil 33 ausgestaltet ist und hierauf dann zunächst die Montageöse 35 und dann die Montageöse 36 folgen.

Analog zu der Variante nach den Fig. 1 und 2 ist der Befestigungsabschnitt 31 bei der Fremdkörperbergeeinrichtung 32 gemeinsam mit dem nicht gezeigten Bergeabschnitt einstückig als textiles Gebilde 40 ausgestaltet, welches durch Verknüpfen des Filaments 10 hergestellt worden ist. Hierbei ist dieses Gebilde 40 in den einen Tragrahmen des Bergeabschnitts, den Verbindungsteil 33 sowie die Montageösen 34 bis 36 des Befestigungsabschnitts 31 ausbildenden Bereichen mit einer höheren Dichte der Maschen ausgebildet, als in einem ein Bergenetz des Bergeabschnitts bildenden Bereich. Dadurch wird auch bei der Fremdkörperbergeeinrichtung 32 im Vergleich zu dem Bergenetz eine höhere Steifigkeit des Tragrahmens und des Befestigungsabschnitts 31 erreicht. Auch im Übrigen entspricht die Fremdkörperbergeeinrichtung 32 sonst der Variante nach den Fig. 1 und 2, so dass hinsichtlich der weiteren Gestaltung auf das zu Fig. 1 und 2 beschriebene Bezug genommen wird.

In Fig. 9 ist das Betätigungselement 23 der endoskopischen Vorrichtung nun dadurch an der Fremdkörperbergeeinrichtung 32 befestigt, indem das Betätigungselement 23 mit seinem Ende durch die Durchgänge 37 bis 39 der Montageösen 34 bis 36 hindurchgefädelt und anschließend mit dem Befestigungsabschnitt 31 verpresst worden ist. Auch die Fremdkörperbergeeinrichtungen 24 und 32 gemäß der Fig. 7 bis 9 können jeweils in analoger Weise zu dem zu Fig. 6 Beschriebenen hergestellt werden, indem im Rahmen der Herstellung mehrere Fremdkörperbergeeinrichtungen 24 bzw. 32 einstückig und in Reihe aufeinanderfolgend durch Verstricken von Filament gefertigt werden. Unterschiedlich ist dabei lediglich die Fertigung des jeweiligen Befestigungsabschnitts 25 bzw. 31, da hier nun jeweils mehrere Montageösen 27 und 28 bzw. 34 bis 36 auszubilden sind, bevor im Folgenden dann der Bergeabschnitt der in Reihe nachfolgenden Fremdkörperbergeeinrichtung 24 bzw. 32 einstückig gefertigt wird.

Mittels der erfindungsgemäßen Ausgestaltungen kann jeweils eine Fremdkörperbergeeinrichtung geschaffen werden, welche sich durch einen niedrigen Herstellungsaufwand auszeichnet und zudem auf zuverlässige Weise in einer endoskopischen Vorrichtung befestigt werden kann.

### Bezugszeichenliste

- 1: Fremdkörperbergeeinrichtung
- 2: Bergeabschnitt
- 3: Befestigungsabschnitt
- 4: Tragrahmen
- 5: Bergenetz
- 6: Verbindungsteil
- 7: Montageöse
- 8: Durchgang
- 9: Gebilde
- 10: Filament
- 11: endoskopische Vorrichtung
- 12: Ende
- 13: Tubus
- 14: Lumen
- 15: Betätigungselement
- 16: Befestigungselement
- 17: Befestigungshaken
- 18: Befestigungselement
- 19: Befestigungselement
- 20: Befestigungshaken
- 21: Befestigungshaken
- 22: Trennstellen
- 23: Betätigungselement
- 24: Fremdkörperbergeeinrichtung
- 25: Befestigungsabschnitt
- 26: Gebilde
- 27: Montageöse
- 28: Montageöse
- 29: Durchgang
- 30: Durchgang
- 31: Befestigungsabschnitt
- 32: Fremdkörperbergeeinrichtung
- 33: Verbindungsteil
- 34: Montageöse
- 35: Montageöse
- 36: Montageöse
- 37: Durchgang
- 38: Durchgang
- 39: Durchgang
- 40: Gebilde

## Patentansprüche

1. Fremdkörperbergeeinrichtung (1; 24; 32) für eine endoskopische Vorrichtung (11), umfassend einen Bergeabschnitt (2), der ein Bergenetz (5) bildet, und einen hieran angrenzenden Befestigungsabschnitt (3; 25; 31), welcher für eine Befestigung der Fremdkörperbergeeinrichtung (1; 24; 32) in der endoskopischen Vorrichtung (11) vorgesehen ist, wobei zumindest das Bergenetz (5) des Bergeabschnitts (2) und der Befestigungsabschnitt (3; 25; 31) gemeinsam einstückig als textiles Gebilde (9; 26; 40) ausgestaltet sind, welches durch Verknüpfen mindestens eines Filaments (10) hergestellt ist, **dadurch gekennzeichnet, dass** das Gebilde (9; 26; 40) bei dem Befestigungsabschnitt (3; 25; 31) mindestens eine Montageöse (7; 27, 28; 34, 35, 36) bildet, an welcher die Befestigung der Fremdkörperbergeeinrichtung (1; 24; 32) in der endoskopischen Vorrichtung (11) herzustellen ist.

2. Fremdkörperbergeeinrichtung (1; 24; 32) nach Anspruch 1, **dadurch gekennzeichnet, dass** das Bergenetz (5) bei dem Bergeabschnitt (2) von einem Tragrahmen (4) umgeben ist.

3. Fremdkörperbergeeinrichtung (1; 24; 32) nach Anspruch 2, **dadurch gekennzeichnet, dass** der Tragrahmen (4) gemeinsam mit dem Bergenetz (5) und dem Befestigungsabschnitt (3; 25; 31) einstückig als das textile Gebilde (9; 26; 40) ausgestaltet ist und im Vergleich zu dem Bergenetz (5) eine höhere Steifigkeit aufweist.

4. Fremdkörperbergeeinrichtung (1; 24; 32) nach Anspruch 3, **dadurch gekennzeichnet, dass** die höhere Steifigkeit des Tragrahmens (4) im Vergleich zu dem Bergenetz (5) verwirklicht ist, indem der Tragrahmen (4) im Vergleich zu dem Bergenetz (5) mit einer höheren Dichte gebildet ist.

5. Fremdkörperbergeeinrichtung (1; 24; 32) nach einem der vorhergehenden Ansprüche, **dadurch gekennzeichnet, dass** der Befestigungsabschnitt (3; 25; 31) im Vergleich zu dem Bergenetz (5) eine höhere Steifigkeit aufweist.

6. Fremdkörperbergeeinrichtung (1; 24; 32) nach Anspruch 5, **dadurch gekennzeichnet, dass** die höhere Steifigkeit des Befestigungsabschnitts (3; 25; 31) im Vergleich zu dem Bergenetz (5) verwirklicht ist, indem der Befestigungsabschnitt (3; 25; 31) im Vergleich zu dem Bergenetz (5) mit einer höheren Dichte gebildet ist.

7. Fremdkörperbergeeinrichtung (1; 32) nach einem der vorhergehenden Ansprüche, **dadurch gekennzeichnet, dass** der Befestigungsabschnitt (3; 31) mit dem Bergeabschnitt (2) über einen länglichen Verbindungsteil (6; 33) gekoppelt ist, welcher den Bergeabschnitt (2) mit der mindestens einen Montageöse (7; 34, 35, 36) verbindet.

8. Fremdkörperbergeeinrichtung (1; 24; 32) nach einem der vorhergehenden Ansprüche, **dadurch gekennzeichnet, dass** das mindestens eine Filament (10) als Monokomponente ausgeführt ist, bevorzugt aus einem thermoplastischen Kunststoff, besonders bevorzugt aus Polyvinylchlorid oder Polyethylen.

9. Fremdkörperbergeeinrichtung (1) nach einem der vorhergehenden Ansprüche, **dadurch gekennzeichnet, dass** das textile Gebilde (9) bei dem Befestigungsabschnitt (3) genau eine Montageöse (7) bildet.

10. Fremdkörperbergeeinrichtung (24; 32) nach einem der Ansprüche 1 bis 8, **dadurch gekennzeichnet, dass** das textile Gebilde (26; 40) bei dem Befestigungsabschnitt (25; 31) mehrere Montageösen (27, 28; 34, 35, 36), bevorzugt genau zwei oder genau drei Montageösen (27, 28; 34, 35, 36), bildet.

11. Endoskopische Vorrichtung (11) mit einem flexiblen Tubus (13), der zumindest ein in seiner Längsrichtung verlaufendes Lumen (14) aufweist, wobei mit radialem Spiel durch das Lumen (14) ein Betätigungselement (15; 23) verläuft, an welchem eine Fremdkörperbergeeinrichtung (1; 24; 32) nach einem oder mehreren der Ansprüche 1 bis 10 angebunden ist.

12. Verfahren zur Herstellung einer Fremdkörperbergeeinrichtung (1; 24; 32) für eine endoskopische Vorrichtung (11), wobei ein ein Bergenetz (5) bildender Bergeabschnitt (2) und ein hieran angrenzender und für eine Befestigung der Fremdkörperbergeeinrichtung (1; 24; 32) in der endoskopischen Vorrichtung (11) vorgesehener Befestigungsabschnitt (3; 25; 31) hergestellt werden, und wobei dabei zumindest das Bergenetz (5) des Bergeabschnitts (2) und der Befestigungsabschnitt (3; 25; 31) gemeinsam einstückig als textiles Gebilde (9; 26; 40) ausgestaltet werden, welches durch Verknüpfen mindestens eines Filaments (10) gebildet wird, **dadurch gekennzeichnet, dass** durch das Gebilde (9; 26; 40) bei dem Befestigungsabschnitt (3; 25; 31) mindestens eine Montageöse (7; 27, 28; 34, 35, 36) gebildet wird, an welcher die Befestigung der Fremdkörperbergeeinrichtung (1; 24; 32) in der endoskopischen Vorrichtung (11) herzustellen ist.

13. Verfahren nach Anspruch 12, **dadurch gekennzeichnet, dass** die Fremdkörperbergeeinrichtung (1; 24; 32) im Weiteren gemäß einem oder mehrerer der Ansprüche 2 bis 10 ausgestaltet wird.

14. Verfahren nach Anspruch 12 oder 13, **dadurch gekennzeichnet, dass** mehrere derartige Fremdkörperbergeeinrichtungen (1; 24; 32) aufeinanderfolgend einstückig als textiles Gebilde (9; 26; 40) durch Verknüpfen des mindestens einen Filaments (10) hergestellt werden, wobei die Fremdkörperbergeeinrichtungen (1; 24; 32) anschließend an Trennstellen (22) voneinander separiert werden.

15. Verfahren nach Anspruch 14, **dadurch gekennzeichnet, dass** die Separierung mittels Heißschneiden vorgenommen wird.
